# EUROPÄISCHE PATENTSCHRIFT

(11) **EP 0 760 654 B1**
(45) Veröffentlichungstag und Bekanntmachung des Hinweises auf die Patenterteilung: **16.08.2001**
(21) Anmeldenummer: 96907439.2
(22) Anmeldetag: 12.03.1996
(51) Int. Cl.: A61K 9/20, A61K 9/14

(54) **VERFAHREN ZUR HERSTELLUNG VON WIRKSTOFFZUBEREITUNGEN IN FORM EINER FESTEN LÖSUNG DES WIRKSTOFFS IN EINER POLYMERMATRIX**
PROCESS FOR PRODUCING ACTIVE AGENT PREPARATIONS IN THE FORM OF A SOLID SOLUTION OF THE ACTIVE AGENT IN A POLYMER MATRIX
PROCEDE PERMETTANT DE PREPARER DES COMPOSITIONS DE PRINCIPE ACTIF SOUS FORME DE SOLUTION SOLIDE DUDIT PRINCIPE ACTIF DANS UNE MATRICE POLYMERE

(30) Priorität: 21.03.1995 DE 19509807
(43) Veröffentlichungstag der Anmeldung: 12.03.1997
(73) Patentinhaber: BASF AKTIENGESELLSCHAFT, 67056 Ludwigshafen (DE)
(72) Erfinder: ROSENBERG, Joerg, D-67158 Ellerstadt (DE); BREITENBACH, Jörg, D-68199 Mannheim (DE)
(86) Internationale Anmeldenummer: EP9601047
(87) Internationale Veröffentlichungsnummer: WO9629061

(56) Entgegenhaltungen:
- WO-A-94/06414

## Beschreibung

Die vorliegende Erfindung beschreibt ein Verfahren zur Herstellung von Wirkstoffzubereitungen, in denen der Wirkstoff molekulardispers verteilt in einer Polymermatrix vorliegt sowie damit hergestellten Wirkstoffzubereitungen.

Schmelzextrusionsverfahren zur Herstellung von Arzneiformen (Tabletten, Pellets, Granulate) sind in der Literatur beschrieben. Insbesondere die Kombination eines Extrusionsschritts mit einem anschließenden Formgebungsschritt macht dieses Verfahren zu einer sehr eleganten, weil einstufigen (und damit kostensparenden) Herstellmethode für Arzneiformen wie z.B. Tabletten (DE-A-1 766 546 und US-A- 4,880,585). In diesen und anderen Literaturstellen (EP-A- 580 860) wird erwähnt, daß sich durch den thermischen Verarbeitungsprozeß bei der Extrusion durch das Schmelzen des Wirkstoffs dieser in Form einer molekulardispersen Verteilung in die ebenfalls geschmolzene Polymerschmelze einarbeiten läßt. Dies äußert sich darin, daß klare, transparente wirkstoffhaltige Schmelzen gebildet werden, die meist auch nach dem Abkühlen dieser Massen auf Raumtemperatur nicht wieder rekristallisieren, sondern ihre molekulardisperse Verteilung beibehalten.

Aus der EP-A-0 661 045 ist die Herstellung von Hydrogel-Zubereitungen mit verzögerter Wirkstofffreisetzung beschrieben, wobei die Zubereitungen neben Hydrogel-bildenden Polymeren auch Salze zur Verbesserung der Auflösung im wässrigen Milieu enthalten können. Solche Hydrogel-Zubereitungen können unter anderem durch Schmelzextrusion hergestellt werden.

Diese Formulierungen werden i.a. als "feste Lösungen" bezeichnet und sind in der Literatur an sich vielfach beschrieben worden (z.B. A.S. Kearny et al.; Int. J. Pharm. **104** (1994), 169-174). Sie wurden aber bisher nur vereinzelt über Schmelzextrusionsverfahren hergestellt. Häufig werden "feste Lösungen" auch durch ein Verfahren hergestellt, bei dem die Komponenten in einem organischen Lösungsmittel aufgelöst werden und das Lösungsmittel dann wieder abgezogen wird. Dies hat den Nachteil, daß zwangsläufig organische Lösungsmittel eingesetzt werden müssen. Außerdem sind diese (mehrstufigen) Verfahren nicht kontinuierlich zu betreiben und die erreichte Homogenität ist nicht befriedigend.

Die molekulardisperse Verteilung eines Wirkstoffs in einem wasserlöslichen Polymer ist eine vielfach beschriebene Methode zur Beschleunigung der Lösungsgeschwindigkeit eines (schwerlöslichen) Wirkstoffs in wäßrigen Medien. In vielen Fällen zeigte sich insbesondere bei schwerlöslichen Wirkstoffen, daß diese nicht oder nur in sehr geringem Umfang nach oraler Gabe aus dem Gastrointestinaltrakt resorbiert werden konnten, da die Lösungsgeschwindigkeit des Wirkstoffs zu langsam war. Nur ein (im wäßrigen Medium) gelöster Wirkstoff kann aber auch resorbiert werden. Mit Hilfe einer molekulardispersen Verteilung des Wirkstoffs in einem wasserlöslichen Polymer gelingt es aber, diesen Vorgang erheblich zu beschleunigen, da aus diesen Formulierungen direkt einzelne Wirkstoff-Moleküle beim Lösevorgang der (wasserlöslichen) Formulierung freigesetzt werden, im Gegensatz zu bisherigen Präparationen, die Wirkstoff-Kristalle enthalten, deren Lösekinetik erheblich langsamer abläuft. Solche "festen Lösungen" führen daher in vielen Fällen zu einer deutlichen Erhöhung des resorbierbaren Wirkstoffanteils, was sich in vielen Fällen zu Verbesserungen der Bioverfügbarkeit eines (schwerlöslichen) Wirkstoffs führt (N. Kondo et al., J. Pharm. Sci. **83** (1994), S. 566-570).

Nicht bei allen Wirkstoffen ist allerdings die Herstellung von "festen Lösungen" möglich.

Die Bildung von "festen Lösungen" durch thermische Verfahren wie z.B. Extrusion (im Gegensatz zu den üblicherweise eingesetzten Verfahren über organische Lösungsmittel) ist an verschiedene Voraussetzungen gebunden, die zumindest teilweise erfüllt sein müssen:
a) der Schmelzpunkt des Wirkstoffs muß so niedrig liegen, daß durch die bei der Extrusion i.a. herrschenden Temperaturen eine intensive Vermischung beider Stoffe in der Schmelze (Wirkstoff- und Polymer) möglich ist, und/oder
b) die Lösungsgeschwindigkeit eines (hochschmelzenden) Wirkstoffs in der Polymerschmelze ist so hoch, daß es zur Ausbildung von "festen Lösungen" trotz der relativ kurzen Verweilzeit im Extruder kommen kann.

Insbesondere der letzte Punkt ist in den meisten Fällen bei hochschmelzenden Wirkstoffen nicht erfüllt, da die Verweilzeiten der Masse im Extruder zu kurz ist (i.a. unter einer Minute). Eine Verlängerung dieser Verweilzeit führt zu (thermischer) Schädigung von Wirkstoff und/oder Polymer und ist daher nicht praktikabel.

Ein erheblicher Teil der bekannten Wirkstoffe besteht jedoch aus ionischen Wirkstoffen, die in Form ihrer Salze eingesetzt werden. Durch die Salzbildung wird in praktisch allen diesen Fällen eine deutliche Erhöhung des Wirkstoff-Schmelzpunkts erreicht, der für die Verarbeitbarkeit der Substanzen nach der konventionellen Technik auch immer erwünscht war. Ausgehend von der nichtionisch vorliegenden Wirkstoff-Form gelingt es, diesen in die molekulardisperse Form zu überführen, da durch den niedrigen Schmelzpunkts dieser dann bei der Extrusion im Gegensatz zur Salzform aufgeschmolzen werden kann und sich dann leicht intensiv mit dem ebenfalls geschmolzenen Polymer bis zur molekulardispersen Verteilung vermischen kann.

Formulierungen, die den Wirkstoff in der nichtionisch vorliegenden Form enthalten, sind allerdings in vielen Fällen nachteilig, da oft nur die entsprechenden Wirkstoff-Salze eine ausreichende Löslichkeit im wäßrigen Milieu aufweisen. Dies bedeutet, daß zwar über die "festen Lösungen" eine schnelle Freisetzung des (molekularen) Wirkstoffs aus der Arzneiform (z.B. Tablette) erfolgt, aber in diesem Fall kein gut wasserlösliches Salz freigesetzt wird, so daß es schnell zu Rekristallisationen kommen kann. Eine ausreichende Wasserlöslichkeit ist aber u.a. zur Ermöglichung einer zufriedenstellenden Resorption unumgänglich.

Der vorliegenden Erfindung lag daher die Aufgabe zugrunde, ein einstufiges, kontinuierlich zu betreibendes Verfahren zur Herstellung von Wirkstoffzubereitungen zur Verfügung zu stellen, durch das Wirkstoffzubereitungen mit einem sehr gut resorbierbaren Wirkstoff erzeugt werden, wobei der Wirkstoff zum einen in Salzform und zum anderen molekulardispers verteilt vorliegt, ohne daß bei der Herstellung Lösungsmittel eingesetzt werden müssen.

Diese Aufgabe wird durch das erfindungsgemäße Verfahren gemäß Anspruch 1 gelöst.

Bevorzugt werden beim erfindungsgemäßen Verfahren wasserlösliche Polymere eingesetzt.

Mit dem erfindungsgemäßen Verfahren gelingt es sehr einfach, das dem Wirkstoff entsprechende "Gegenion" in Form eines "passenden" (i.a. anorganischen) Salzes ebenfalls in die zu extrudierende (Pulver-)mischung zuzugeben. Hierdurch ist es erstmals möglich, ausgehend von dem nichtionischen Wirkstoff (z.B. deprotoniertes Amin oder protonierte Carbonsäure) durch den thermischen Verarbeitungsprozeß (Extrusion) eine intensive Vermischung beider Schmelzen zu erreichen (Wirkstoff und Polymer), und gleichzeitig oder im Anschluß daran durch Zugabe eines sauren bzw. alkalischen, zusätzlichen Salzes die gewünschte ionische Wirkstoffkomponente noch während des Extrusionsprozesses, quasi in situ, zu erzeugen.

Mit diesem neuen Verfahrensprinzip gelingt es erstmals, Wirkstoff-Salze in wasserlöslichen Polymeren molekulardispers über den "Umweg" der nichtionischen, niedrigschmelzenden Form zu verteilen und sehr homogene Verteilungen zu erreichen. Bisher waren diese Präparationen über den rein "thermischen" Prozeß der Extrusion infolge ihres zu hohen Schmelzpunkts nicht in dieser Form herstellbar, so daß auf die üblichen Lösungsmittel-Verfahren zurückgegriffen werden mußte.

Als Wirkstoffe sind z.B. einsetzbar:

Acetylcystein, Acetylsalicylsäure, Ambroxol, Atenolol, Biperiden, Clävulinsäure, Cromoglycinsäure, Diltiazem, Dopamin, Ephedrin, Flurbiprofen, Ibuprofen, Lidocain, Metoprolol, Methylephedrin, Naftidrofuryl, Nicotinsäure, Pantothensäure, Propafenon, Propanolol, Pseudoephedrin, Salicylsäure, Sotalol, Valproinsäure, Verapamil.

In Einzelfällen kann das neue Verfahren auch dazu verwendet werden, Wirkstoffe, die bisher vorwiegend in ihrer nichtionischen Form verabreicht wurden, durch gezielte Salzbildung besser bioverfügbar zu machen. Ein Beispiel dafür ist das Ibuprofen, ein Wirkstoff, der eine (protonierte) Carbonsäuregruppe trägt. Das Ibuprofen wird zur Schmerztherapie eingesetzt, die allgemein einen schnellen Wirkungseintritt verlangt (z.B. Kopfschmerztablette). Eine schnelle Wirkungsentfaltung setzt aber voraus, daß der Wirkstoff nach oraler Gabe (z.B. nach Einnahme einer Tablette) schnell gelöst wird, so daß die folgende Resorption schnell ablaufen kann. Hier sind also Präparationen mit schneller Lösegeschwindigkeit des Wirkstoffs vorteilhaft. "Feste Lösungen" auf Basis des nichtionisch vorliegenden Wirkstoffs, die allein schon zur schnellen Solubilisierung beitragen, sind in der Literatur beschrieben (EP-A- 580 860). Diese Präparationen weisen aber Nachteile auf, da der Wirkstoff liegt in der schlecht wasserlöslichen, nichtionischen Form vorliegt, wodurch zwar die Herstellbarkeit der "festen Lösungen" gegeben ist. Andererseits werden die besser wasserlöslichen Salze zur "schnellen" Therapie benötigt.

Diese bisher bekannten Formulierungen auf Basis "fester Lösungen" können durch Anwendung des erfindungsgemäßen Verfahrens über gezielte Salzbildungen verbessert werden, da die Ibuprofen-Salze besser wasserlöslich sind als der nichtionisch vorliegende Wirkstoff. Dadurch wird die Geschwindigkeit der Wirkstoffabgabe aus der Arzneiform (z.B. Tablette) erhöht, wie durch die hierfür gängigen in-vitro Freisetzungsmodelle leicht gezeigt werden kann.

Ibuprofen-Präparationen, die den Wirkstoff als Salz enthalten, sind in der Literatur beschrieben (z.B. Lysinat- oder Natrium-Salze), und insbesondere beim Lysinat-Salz ist der schnellere Wirkungseintritt bei diesen Präparationen im Vergleich zu Formulierungen, die den nichtionischen Wirkstoff enthalten, klinisch belegt (G. Geisslinger et al., Drug Investigation **5** (1993), S. 239 ff). Das neue, erfindungsgemäße Verfahren ermöglicht es nun, solche Formulierungen (z.B. Lysinate) direkt aus dem nichtionisch vorliegenden Wirkstoff bei der Arzneimittel-Herstellung zu bilden, ohne daß zuvor in einem separaten Schritt das jeweilige Wirkstoff-Salz erst synthetisiert werden müßte. Im Fall des Ibuprofens beispielsweise zeigte es sich, daß es durch Zugabe von z.B. Natriumacetat gelang, das Ibuprofen in einer Polymermatrix aus Vinylpyrrolidon-Vinylacetat-Copolymer in Form einer klaren Schmelze als "feste Lösung" zu solubilisieren.

Die Herstellbarkeit von Formulierungen nach dem erfindungsgemäßen Verfahren hängt entscheidend davon ab, wie gut der Wirkstoff in dem wasserlöslichen Polymer löslich ist. Besonders bevorzugt sind daher naturgemäß wasserlösliche Polymere, die für eine Vielzahl von Wirkstoffen als "Lösungsmittel" bekannt und beschrieben sind. Hierzu zählen besonders bevorzugt die Polyvinylpyrrolidone (Homo- und Copolymere), wie sie auch schon in der DE 1 766 546 und in der US 4,880,585 erwähnt werden. Bevorzugt sind ferner Polyethylenglycole und Polyethylenoxide, sowie Hydroxyalkylcellulosen wie z.B. Hydroxypropylcellulose. Bevorzugt sind ferner Cellulosederivate, die Carboxyl-Substituenten tragen, wie z.B. Celluloseacetat-phthalat (CAP), die im sauren Medium (Magensaft, pH 1) unlöslich sind und sich erst im Dünndarm bei den dort höheren pH-Werten (pH 6-7) auflösen. Die Verwendung dieser Polymere bewirkt die bekannte sog. "Magensaftresistenz" derartiger Präparationen durch das Fehlen der Wirkstoff-Freisetzung im Magen, wodurch sich die Möglichkeit ergibt, z.B. säurelabile Wirkstoffe intakt in den Dünndarm gelangen zu lassen, wo sie unverändert resorbiert werden können (vgl. R. Voigt; Lehrbuch der pharmazeutischen Technologie; Verlag Chemie 1984, Seite 209 ff). Bevorzugt sind ferner Methacrylsäure-Polymere, insbesondere Copolymere aus Methylmethacrylat und Ethylacrylat, die zusätzlich bis ca. 15% Trimethylammoniummethylmonoacrylat-chlorid enthalten. Alle diese Polymere können allein oder in Mischungen vorliegen, wodurch sich die Eigenschaften der so gebildeten Formulierungen gezielt beeinflussen lassen. Entscheidend für die Verwendbarkeit ist, daß der gewählte Wirkstoff in der Polymermatrix ausreichend löslich ist. Im allgemeinen kann dies an dem Aussehen der den Extruder verlassenden wirkstoffhaltigen Schmelze rein visuell schon abgeschätzt werden. "Feste Lösungen" geben sich durch klare, absolut transparente Schmelzen zu erkennen, die auch nach dem Abkühlen auf Raumtemperatur ihre optische Transparenz beibehalten. Durch physikalische Methoden wie z.B. Differential-Thermoanalyse kann dann weiterhin zweifelsfrei das Fehlen kristalliner Wirkstoffreste nachgewiesen werden.

Das erfindungsgemäße Verfahren ist bevorzugt für solche Wirkstoffe einsetzbar, die zur Salzbildung befähigt sind, aber so hohe Schmelzpunkte in Form ihrer bevorzugt eingesetzten Salze aufweisen, daß unter den bisher bekannten Extrusionsverfahren keine Herstellung von Formulierungen mit molekulardispersen Verteilungen des Wirkstoffs gelang. Die üblichen Extrusionstemperaturen liegen im Bereich von 60 bis 160 °C, bevorzugt bei 80 bis 140 °C. Der Wirkstoff sollte daher in der nichtionischen Form einen Schmelzpunkt besitzen, der bevorzugt maximal 140 °C beträgt. Deutlich niedrigere Schmelzpunkte sind möglich; auch bei Raumtemperatur flüssige oder ölige Wirkstoffe können verwendet werden. In diesen Fällen kann entweder ein "Aufziehen" auf feste Trägerstoffe vorgeschaltet werden (z.B. Lactose, Kieselgele o.ä.), oder aber der Wirkstoff wird flüsssig über Pumpen direkt in den Extruder nach den in der Kunststofftechnologie bekannten Verfahren, z.B. über Hochdruckpumpen, zudosiert ("Seitendosierung").

Als "Salzkomponente" kommen verschiedenste organische und anorganische Salze in Betracht. Bevorzugt für anionische Wirkstoffe (z.B. Carboxylgruppen-tragende Wirkstoffe) sind Natriumsalze organischer Carbonsäuren wie z.B. Natriumacetat, -succinat, -aspartat, -maleat, -glycinat, -lysinat, -citrat, -lactat, -gluconat, -tartrat, sowie Natriumsalze anorganischer Verbindungen wie z.B. Natriumhydrogenphosphat, Natriumdihydrogenphosphat und Natriumphosphat. Für kationische Wirkstoffe (z.B. basische Aminogruppen tragende Wirkstoffe) sind "saure" Salze bevorzugt, wie z.B. Hydrochloride von Aminosäuren, aber auch Carbonsäuren wie z.B. Essigsäure, Bernsteinsäure, Asparaginsäure, Äpfelsäure, Glycin, Lysin, Citronensäure, Milchsäure, Gluconsäure und Weinsäure.

Die Menge der Salzkomponente bestimmt sich im wesentlichen durch die Menge des nichtionischen Wirkstoffs.

Den erfindungsgemäßen Formulierungen können verschiedene weitere, pharmazeutisch gebräuchliche Hilfsstoffe zugesetzt werden, um z.B. die Verarbeitbarkeit oder andere Eigenschaften gezielt zu beeinflussen. Zu diesen Hilfsstoffen zählen z.B. Füllstoffe (z.B. Lactose, Mannit), Schmiermittel (z.B. Mono-, Di-und Triglyceride, sowie Salze von Fettsäuren), Formtrennmittel (z.B. Lecithin), Weichmacher (z.B. Fettsäuren, Fettalkohole oder Triethylcitrat), Stabilisatoren (z.B. Antioxidantien wie Ascorbinsäure oder Butylhydroxytoluol), Farbstoffe oder Pigmente (z.B. Eisenoxidpigmente), Sprengmittel (z.B. quervernetztes Polyvinylpyrrolidon, mikrokristalline Cellulose), Konservierungsmittel (z.B. Sorbinsäure), oder Geschmacksstoffe (z.B. Aromen, Süßstoffe). Eine wichtige Voraussetzung für die Auswahl dieser Hilfsstoffe ist eine ausreichende (thermische) Beständigkeit bei dem gewählten Verarbeitungsprozeß (Extrusion). Weiterhin muß gewährleistet sein, daß durch die Anwesenheit weiterer Hilfsstoffe die zur Solubilisierung des Wirkstoffs minimal notwendige Polymermenge nicht unterschritten wird, da es sonst zur Rekristallisation des Wirkstoffs in der Matrix kommen kann, was sich durch Eintrübung bemerkbar macht.

Die Herstellung der erfindungsgemäßen Präparationen erfolgt in den an sich bekannten Verfahren, vorzugsweise in Ein- oder Doppelschneckenextrudern, wobei hier insbesonders gleichläufig drehende Doppelschneckenextruder wegen ihrer intensiveren Mischwirkung besonders bevorzugt sind. Eine Formgebung der wirkstoffhaltigen Schmelzen kann auf vielfältige Weise erfolgen. Eine direkte Schmelze-Kalandrierung z.B. zu Tabletten ist gemäß EP-A 240 906 möglich. Die Herstellung von Pellets durch Zerkleinerung dünner Extrudat-Stränge mit Hilfe rotierender Messer gemäß DE-A 38 30 355 ist ebenfalls möglich. Beide Verfahren bieten den Vorteil, daß sie kontinuierlich und direkt im Anschluß an den Extrusionsschritt erfolgen können (quasi "on-line"). Es ist aber auch möglich, die extrudierte Schmelze abkühlen zu lassen, und erst dann weitere Verfahrensschritte zur Formgebung anzuschließen, z.B. eine Mahlung zu Granulaten, die als Instant-Trinkpulver dienen können, oder die in Hartgelatine-Steckkapseln abgefüllt oder zu Tabletten verpreßt werden können. Die erfindungsgemäßen Zubereitungen werden i.a. als Arzneimittel eingesetzt. Es ist aber ebenso möglich, Wirkstoffe , die z.B. zur Behandlung von Pflanzenkrankheiten und zur Insektenvertilgung bekannt sind, nach dem erfindungsgemäßen Verfahren zu verarbeiten. Wirkstoffe im Sinne des erfindungsgemäßen Verfahrens sind auch Vitamine und Mineralstoffe (z.B. "Spurenelemente").

Die folgenden Beispiele erläutern die Erfindung.

### Beispiel 1 (Vergleichsbeispiel)

Eine Pulvermischung, bestehend aus 20,0 Gew.-% Ibuprofen (nichtionisch) und 80 Gew.-% Vinylpyrrolidon-Vinylacetat-Copolymer (Kollidon VA-64 (BASF)) wurden in einem Zweischneckenextruder (ZSK-30, Fa. Werner und Pfleiderer) zu einer klaren, transparenten Schmelze extrudiert. Die Schmelze wurde nach dem in der US 4,880, 585 bekannten Verfahren direkt nach Verlassen des Extruders zu etwa 1000 mg schweren Oblong-Tabletten mit Hilfe eines Formkalanders gepreßt. Folgende Extrusionsbedingungen wurden eingestellt:

| | |
|---|---|
| Temperatur Schuß 1 | 50 °C |
| Temperatur Schuß 2 | 85 °C |
| Temperatur Schuß 3 | 125 °C |
| Temperatur Schuß 4 | 100 °C |
| Temperatur Schuß 5 | 100 °C |
| Temperatur Kopf | 90 °C |
| Temperatur Düsen | 80 °C |

Die Wirkstoff-Freisetzung aus diesen Tabletten wurde mit Hilfe der USP-Paddle-Methode

in 900 ml Phosphatpuffer (pH 7,5) bei 37 °C und einer Rührerdrehzahl von 100 Upm bestimmt. Die Konzentrationsbestimmungen des Wirkstoffs in der Prüflösung erfolgte über UV-Spektroskopie.

Folgende Wirkstoff-Freisetzungen wurden erhalten:
- nach 10 Minuten: 35,9 %
- nach 20 Minuten: 65,7 %
- nach 30 Minuten: 84,3 %
- nach 60 Minuten: 100 %

### Beispiel 2

Eine Pulvermischung, bestehend aus 20, 0 Gew.-% Ibuprofen (nichtionisch), 75,0 Gew.-% Kollidon VA-64 und 5,0 Gew.-% Natriumacetat-Trihydrat wurde unter folgenden Verarbeitungsbedingungen in einem Zweischneckenextruder (ZSK-30, Fa.Werner und Pfleiderer) zu einer klaren, transparenten Schmelze extrudiert:

| | |
|---|---|
| Temperatur Schuß 1 | 50 °C |
| Temperatur Schuß 2 | 80 °C |
| Temperatur Schuß 3 | 110 °C |
| Temperatur Schuß 4 | 90 °C |
| Temperatur Schuß 5 | 90 °C |
| Temperatur Kopf | 80 °C |
| Temperatur Düsen | 80 °C |

Die Bestimmung der Wirkstoff-Freisetzung der analog Beispiel 1 erhaltenen ca. 1000 mg schweren Oblong-Tabletten erfolgte mit der identischen Methode:
- nach 10 Minuten: 48,1 %
- nach 20 Minuten: 77,8 %
- nach 30 Minuten: 95,2 %
- nach 60 Minuten:100 %.

## Patentansprüche

1. Verfahren zur Herstellung von Wirkstoffzubereitungen in Form einer festen Lösung des Wirkstoffs, wobei der Wirkstoff in Salzform vorliegt, in einer Polymermatrix, dadurch gekennzeichnet, daß der zur Salzbildung befähigte Wirkstoff in nichtionischer Form zusammen mit einem Polymer und einem Salz schmelzextrudiert wird.

2. Verfahren nach Anspruch 1, dadurch gekennzeichnet, daß als Polymer ein wasserlösliches Polymer eingesetzt wird.

3. Verfahren nach Anspruch 2, dadurch gekennzeichnet, daß als wasserlösliches Polymer Polyvinylpyrrolidon, ein Vinylpyrrolidon-Vinylacetat-Copolymer oder Hydroxyalkylcellulose eingesetzt wird.

## Claims

1. A process for producing active substance compositions in the form of a solid solution of the active substance, the active substance being in salt form, in a polymer matrix, which comprises melt extrusion of the active substance capable of salt formation in nonionic form together with a polymer and a salt.

2. A process as claimed in claim 1, wherein a water-soluble polymer is employed as polymer.

3. A process as claimed in claim 2, wherein polyvinylpyrrolidone, a vinylpyrrolidone/vinyl acetate copolymer or hydroxyalkylcellulose is employed as water-soluble polymer.

## Revendications

1. Procédé pour préparer des compositions de substances actives à l'état de solutions solides de la substance active, laquelle est à l'état de sel, dans une gangue polymère, caractérisé par le fait que l'on soumet la substance active salifiable mais sous forme non-ionique à extrusion à l'état fondu avec un polymère et un sel.

2. Procédé selon la revendication 1, caractérisé par le fait que le polymère utilisé est un polymère soluble dans l'eau.

3. Procédé selon la revendication 2, caractérisé par le fait que le polymère soluble dans l'eau est une polyvinylpyrrolidone, un copolymère vinylpyrrolidone-acétate de vinyle ou une hydroxyalkylcellulose.
